# EUROPEAN PATENT SPECIFICATION

(11) **EP 1 257 306 B1**
(45) Date of publication and mention of the grant of the patent: **15.04.2009**
(21) Application number: 01916085.2
(22) Date of filing: 12.02.2001
(51) Int. Cl.: A61L 27/36, A61F 2/28

(54) **ASSEMBLED IMPLANT**
GEBAUTES IMPLANTAT
IMPLANT CONSTITUE

(30) Priority: 10.02.2000 US 181622 P; 12.05.2000 US 123227
(43) Date of publication of application: 20.11.2002
(73) Proprietor: RTI Biologics, Inc., Alachua, FL 32615 (US)
(72) Inventor: BIANCHI, John, R., Alachua, FL 32615 (US); MILLS, C., Randal, Alachua, FL 32615 (US); GORHAM, P., J., Alachua, FL 32615 (US); ESCH, Michael, Alachua, FL 32615 (US); CARTER, Kevin, C., Alachua, FL 32615 (US); COLEMAN, Pat, Alachua, FL 32615 (US); ROSS, Kevin, Alachua, FL 32615 (US); RAMBO, Harry, W., Alachua, FL 32615 (US); JONES, Darren, G., Alachua, FL 32615 (US); BUSKIRK, Dayna, Alachua, FL 32615 (US)
(74) Representative: Samuels, Lucy Alice
(86) International application number: PCT/US2001/004510
(87) International publication number: WO 2001/078798

(56) References cited:
- WO-A-00/29037
- WO-A-00/40177
- US-A- 5 676 700
- US-A- 5 899 939
- US-A- 6 025 538
- GIE G A ET AL: "CONTAINED MORSELIZED ALLOGRAFT IN REVISION TOTAL HIP ARTHROPLASTY SURGICAL TECHNIQUE" ORTHOPEDIC CLINICS OF NORTH AMERICA,GB,W.B.SAUNDERS CO., LONDON, vol. 24, no. 4, October 1993 (1993-10), pages 717-725, XP000893104 ISSN: 0030-5898

## Description

### FIELD OF THE INTENTION

This invention relates to implants and methods for their preparation wherein components of the implant are assembled from constituent pieces to produce a complete implant.

### BACLKGROUND OF THE INVENTION

In the field of medicine, there has been an increasing need to develop implant materials for correction of biological defects. Particularly in the field of orthopedic medicine, there has been the need to replace or correct bone, ligament and tendon defects or injuries. As a result, there have emerged a number of synthetic implant materials, including but not limited to metallic implant materials and devices, devices composed in whole or in part from polymeric substances, as well as allograft, autograft, and xenograft implants. It is generally recognized that for implant materials to be acceptable, they must be pathogen-free, and must be biologically acceptable. Generally, it is preferable if the implant materials may be remodeled over time such that autogenous bone replaces the implant materials. This goal is best achieved by utilizing autograft bone from a first site for implantation into a second site. However, use of autograft materials is attended by the significant disadvantage that a second site of morbidity must be created to harvest autograft for implantation into a first diseased or injured site. As a result, allograft and xenograft implants have been given increasing attention in recent years. However, use of such materials has the disadvantage that human allograft materials are frequently low in availability and are high in cost of recovery, treatment and preparation for implantation. By contrast, while xenograft implant materials, such as bovine bone, may be of ready availability, immunological and disease transmission considerations imply significant constraints on the ready use of such materials.

In view of the foregoing considerations, it remains the case that there has been a long felt need for unlimited applies of biologically acceptable implant materials for repair of bone and other defects or injuries. This invention provides a significant advance in the art, and largely meets this need, by providing graft implants assembled from component parts.

In recent months, there have appeared several patents and patent publications which address similar or identical considerations to those to which the present invention disclosure is directed.

WO 00140177 discloses a composite bone graft for Implantation into a patient, which includes two or more connected, discrete bone portions and one or more biocompatible connectors which hold together discrete base portions to form the composite bone graft.

US 5,899,939 discloses a bone-derived implant made up of one or more layers of fully mineralized or partially demineralized cortical bone and, optionally, one or more layers of some other material. The layers are assembled to provide an implant exhibiting good overall load-supporting properties.

US 6,025,538 discloses a composite allograft bone device comprising a first bone member body with a face that includes a plurality of intersecting grooves and a second bone member body defining a face that includes a plurality of angularly intersecting grooves. The projections on the second face fit into grooves cut in the first face allowing the two bodies to be mated together.

### Summary of the Invention

According to a first aspect, the present invention provides a graft implant comprising segments of cortical bone, cancellous bone, cortical-cancellous bone, or a combination thereof, pinned to each other by means of cortical bone pins, wherein said graft implants is in the form of a tapered dowel bearing external threading, and wherein said segments are in the form of bone disks.

According to a second aspect, the present invention provides a kit for manufacture of the graft implant of the first aspect of the invention, the kit comprising assemblable implant segment of the cortical bone, cancellous bone, cortical-cancellous bone, or a combination thereof, and assembly pins of cortical bone.

### BRIEF DESCRIPTION OF THE DRAWINGS

Attached to this invention disclosure are sketches which demonstrate the assembled implants according to this invention.

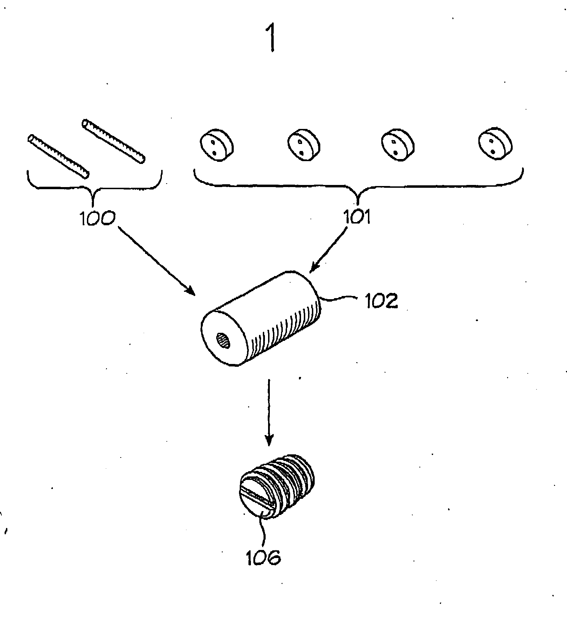

Figure 1 is a flow chart showing the formation of component parts of an assembled implant according to this invention, from which assembled implants and a kit comprising these parts may be formed according to the disclosure of this invention.

### DETAILED DESCRIPTION OF THE PREFERRED EMBODIMENTS

Currently, autograft, allograft and xenograft products are produced as solid, continuous materials. For example, bone dowels, Smith-Robinson cervical spine implants, iliac crest grafts, and the like are harvested and machined from single, continuous pieces of bone. The present invention provides implants assembled from smaller pieces of graft materials to form a larger graft implant product. As a result, increased utilization of valuable implant materials is achieved, thereby more effectively meeting the ever-increasing demands for graft implant materials. In addition greater flexibility is achieved. Orthopedic surgeons may be provided with kits of assemblable parts which may be formed in the course of a surgical procedure to precisely meet the needs of a given patient or procedure. In another aspect of this invention, existing graft products may be strengthened or reinforced by assembly of different types of graft materials into an assembled product. The reinforced product is a tapered dowel into which is inserted one or more reinforcing pins of cortical bone. As a result, those skilled in the art will understand from this disclosure that a plurality of bone portions may be layered to make a complete graft implant. Furthemore, the assembled implants or the component pieces which are combined to form the assembled implant may be pre-treated or treated after assembly to incorporate any desired biologically active materials. Thus, for example, in the assembled bone dowel implant according to this invention, the assembled bone dowel comprises segments of cortical bone in the form of bone disks pinned to each other by means of cortical bone pins. Prior to assembly or after assembly, the graft materials may be soaked, infused, impregnated, coated or otherwise treated with bone morphogenetic proteins (BMP's), antibiotics, growth factors, nucleic acids, peptides, or a combination thereof.

The assembled segments of bone disks are affixed to each other by means of pins made from cortical bone. These pins are machined in a CNC lathe or the like to appropriate dimensions and are then threaded into mating holes tapped in the segments to be assembled, or are pressed into drilled holes through adjacent pieces to be assembled by a pneumatic press or the like. In this fashion, very strong and tightly fitted segments of implant materials may be joined and implanted.

As noted above, the implant according to this invention comprises an assembled tapered dowel, harvested from the iliac crest or another suitable site.

As is known in the art, due to the wafer-like structure of cancellous bone, such grafts have low load-bearing characteristics. There exist reports in the literature of instances of extrusion, expulsion or collapse of iliac crest wedges, Cloward Dowels, and the like when utilized, for example, in spinal fusions. Nonetheless, use of cancellous bone is preferable over use of cortical bone implants, since cancellous bone is more osteoconductive than cortical bone. According to this invention, a tapered dowel is reinforced by insertion therein of cortical bone pins. According to this invention, cortical implants may also be reinforced by insertion therein of cortical bone pins, including when an assembled implant is prepared comprising different segments of cortical bone, cancellous bone or both. Insertion of the reinforcing pins provides an implant with multiple load-bearing pillars. The pins may be made to protrude from the surface of the implant to engage with inferior, superior or both surfaces of bone between which the implant is inserted. Thus, in a spinal implant, pin protrusions may be employed to created contact between the implant and the vertebral bodies, thus preventing extrusion and reinforcing a secure fit of the implant between adjacent vertebrae. We have, surprisingly, found that cortical pins of about 4.5 mm in diameter may each support a load of up about 2700 newtons (160 Mpa). Thus multiple pins may be inserted into an implant to produce a load-bearing capacity of known proportions (e.g. 10,000 newtons by insertion of five pins).

A further advantage of this invention is that it permits use of tissues that are not currently amenable to standard autograft, allograft for xenograft harvesting and processing procedures, such as ribs, metatarsal bone and the like. In addition, useful implant materials may be harvested and produced from otherwise un-useable donor tissues. In addition, due to the different nature of various segments of bone disks that are incorporated into the assembled, reinforced implants of this invention, various shaping methods aside from CNC lathe or other known procedures may be applied to different segments of the implant. Thus, a cancellous segment of bone implant may be compression molded, and then affixed to other segments of cortical or cancellous bone machined according to different or similar principles. In addition, due to the ability provided by this invention to assemble implant pieces, implants of unusual sizes and dimensions may be prepared and machined.

In view of the present disclosure, it will be appreciated that this invention provides dowel shaped implants comprising assembled segments, between about two to about ten segments, pinned together by one or more cortical bone pins. Such implants are prepared by harvesting disks of conical bone, drilling and optionally tapping holes therein, and inserting shafts of cortical pins therethrough, or therein, optionally by threading portions thereof for torquing into optionally tapped holes. The thus produced dowels are tapered. As shown in Figure 1, the external surface of the tapered dowel is threaded.

Referring now to figure 1, there is shown a flow-chart representing the elements that are assembled according to this invention. Cortical bone pins 100 are used to assemble a series of bone disks 101 into a pre-part 102 which is then machined into tapered dowels bearing external threading 106. From this figure, it will be appreciated that a central concept relevant to the present invention is the ability to machine smaller parts of tissue, specifically bone tissue, such as cortical bone, cancellous bone, cortical-cancellous bone, portions of which may be demineralized, and assemble these portions of tissue using cortical bone pins. The assembled tissue pieces are machined prior to assembly, and then, upon assembly, a complete implant is ready for implantation. In figure 1, the pre-machined tissue of the invention is disclosed, which may conveniently be included in a kit for use as needed by an orthopedic surgeon. Thus, for example, where a particular implant of specific dimensions is required, the surgeon is able to select pre-shaped implant segments to fill a particular geometric space and shape in the spine of an implant recipient. Numerous permutations and combinations of implant pieces for assembly are possible, based on the pre-machined assemblable implant pieces included in such a kit, and those skilled in the art will appreciate that the stalled orthopedic surgeon will be able to create implants as needed when supplied with such a kit. Thus, a preferred kit includes disks of bone, cortical bone, cancellous bone, allograft or xenograft, also referred to herein as "washers" or "doughnuts" such that a center hole is provided for press-fitting or screwing on of the disks to a cortical bone or synthetic or metallic shaft or pin. The disks may be demineralized, mineralized, or partially demineralized. Also desirable in such a kit are plugs of cortical bone, cancellous bone, or cortical-cancellous bone, including at least one through hole, and optionally more than one such through hole, for insertion of pins therethrough. Ovals, squares, rectangles and irregular shapes may also be provided in certain kits for specific applications. It will further be appreciated based on the present disclosure, that inclusion of a bone paste may be beneficial for filling any voids that remain, and to implant with the assembled implant, osteogenic material, (i.e. osteoconductive material, Osteoinductive material, or both, as well as material that assists in adhering the implant to the site of implantation). Further, a molded implant may be combined with the assembled implant of this invention.

## Claims

1. A graft implant comprising segments of cortical bone, cancellous bone, cortical-cancellous bone, or a combination thereof, pinned to each other by means of cortical bone pins, wherein said graft implant is in the form of a tapered dowel bearing external threading, and wherein said segments are in the form of bone disks.

2. A kit for manufacture of a graft implant according to claim 1, comprising assemblable implant segments of the cortical bone, cancellous bone, cortical-cancellous bone, or a combination thereof, and assembly pins of cortical bone.

3. The graft implant according to claim 1, wherein the implant is pre-treated or treated after assembly to incorporate a biologically active material.

4. The graft implant according to claim 1 or 3, comprising said cancellous bone which is reinforced by insertion therein of cortical bone pins.

5. The graft implant according to any of claims 1, 3 or 4, wherein, prior to assembly or after assembly, the graft materials are soaked, infused, impregnated, coated or otherwise treated with a bone morphogenic protein (BM), antibiotic, growth factor, nucleic acid, peptide, or a combination thereof.

6. The graft implant according to any of claims 1, 3, 4 or 5, which is a spinal implant,

7. The graft implant according to any of claims 1, 3, 4 or 5, comprising a cancellous bone segment that has been compression molded.

8. The graft implant according to any of claims 1, 3, 4 or 5, comprising a plurality of bone portions layered to from a graft unit, and one or more cortical bone pins for holding together said graft unit, wherein said graft implant does not comprise an adhesive.

## Patentansprüche

1. Graft-Implantat, umfassend Segmente von kortikalem Knochen, spongiösem Knochen, kortikalem-spongiösem Knochen oder einer Kombination davon, die miteinander mithilfe von kortikalen Knochenstiften befestigt sind, wobei das Graft-Implantat in Form eines sich verjüngenden Dübels, der ein Außengewinde trägt, vorliegt und wobei die Segmente in Form von Knochenscheiben vorliegen.

2. Kit zur Herstellung eines Graft-Implantats nach Anspruch 1, umfassend zusammensetzbare Implantatsegmente von dem kortikalen Knochen, spongiösen Knochen, kortikalen-spongiösen Knochen oder einer Kombination davon und Verbindungsstifte aus kortikalem Knochen.

3. Graft-Implantat nach Anspruch 1, worin das Implantat vorbehandelt oder nach der Zusammenstellung behandelt ist, um ein biologisch aktives Material einzubauen.

4. Graft-Implantat nach Anspruch 1 oder 3, umfassend den spongiösen Knochen, der durch Einsetzung von kortikalen Knochenstiften darin verstärkt ist.

5. Graft-Implantat nach irgendeinem der Ansprüche 1, 3 oder 4, wobei die Graftmaterialien vor der Zusammenstellung oder nach der Zusammenstellung mit einem Knochen-morphogenetischen Protein (BMP), Antibiotikum, Wachstumsfaktor, Nucleinsäure, Peptid oder einer Kombination davon getränkt, infundiert, imprägniert, beschichtet oder auf andere Weise behandelt werden.

6. Graft-Implantat nach irgendeinem der Ansprüche 1, 3, 4 oder 5, das ein spinales Implantat ist.

7. Graft-Implantat nach irgendeinem der Ansprüche 1, 3, 4 oder 5, das ein spongiöses Knochensegment umfasst, das formgepresst worden ist.

8. Graft-Implantat nach irgendeinem der Ansprüche 1, 3, 4 oder 5, umfassend eine Mehrheit von Knochenteilen, die zur Bildung einer Grafteinheit geschichtet sind, und einen oder mehrere kortikale Knochenstifte zum Zusammenhalten der Grafteinheit, wobei das Graft-Implantat keinen Klebstoff umfasst.

## Revendications

1. Implant pour greffe, comprenant des segments d'os cortical, d'os spongieux, d'os cortical-spongieux, ou d'une combinaison de ceux-ci, attachés l'un à l'autre à l'aide de broches en os cortical, où ledit implant pour greffe a la forme d'une cheville effilée portant un filetage externe, et où lesdits segments ont la forme de disques osseux.

2. Kit pour la préparation d'un implant pour greffe selon la revendication 1, comprenant des segments d'implant assemblables d'os cortical, d'os spongieux, d'os cortical-spongieux, ou d'une combinaison de ceux-ci, et des broches d'assemblage en os cortical.

3. Implant pour greffe selon la revendication 1, où l'implant est prétraité ou traité après assemblage pour incorporer un matériau biologiquement actif.

4. Implant pour greffe selon la revendication 1 ou 3, comprenant ledit os spongieux, qui est renforcé par insertion de broches d'os cortical dans celui-ci.

5. Implant pour greffe selon l'une quelconque des revendications 1, 3 ou 4, où avant assemblage ou après assemblage, les matériaux de la greffe sont trempés, infusés, imprégnés, revêtus ou traités d'une autre manière avec une protéine morphogénique osseuse (BM), un antibiotique, un facteur de croissance, un acide nucléique, un peptide, ou une combinaison de ceux-ci.

6. Implant pour greffe selon l'une quelconque des revendications 1, 3, 4 ou 5, qui est un implant vertébral.

7. Implant pour greffe selon l'une quelconque des revendications 1, 3, 4 ou 5, comprenant un segment d'os spongieux, qui a été moulé par compression.

8. Implant pour greffe selon l'une quelconque des revendications 1, 3, 4 ou 5, comprenant une série de parties d'os, superposées pour former une unité de greffe, et une ou plusieurs broches d'os cortical pour maintenir ensemble ladite unité de greffe, ledit implant pour greffe ne comprenant pas d'adhésif.
